(19) Europäisches Patentamt
European Patent Office
Office européen des brevets

(11) **EP 2 069 016 B1**

(12) **EUROPEAN PATENT SPECIFICATION**

(45) Date of publication and mention of the grant of the patent:
**11.07.2012 Bulletin 2012/28**

(21) Application number: **07818614.5**

(22) Date of filing: **02.10.2007**

(51) Int Cl.:
***A61N 5/10*** *(2006.01)*

(86) International application number:
**PCT/EP2007/008534**

(87) International publication number:
**WO 2008/040522 (10.04.2008 Gazette 2008/15)**

(54) **IMPROVEMENTS OF TREATMENT PLANNING SYSTEMS**

VERBESSERUNG VON BEHANDLUNGSPLANUNGSSYSTEMEN

AMÉLIORATIONS DES SYSTÈMES DE PLANIFICATION DE TRAITEMENT

(84) Designated Contracting States:
**AT BE BG CH CY CZ DE DK EE ES FI FR GB GR HU IE IS IT LI LT LU LV MC MT NL PL PT RO SE SI SK TR**

(30) Priority: **03.10.2006 GB 0619493**

(43) Date of publication of application:
**17.06.2009 Bulletin 2009/25**

(73) Proprietor: **Elekta AB (publ)**
**103 93 Stockholm (SE)**

(72) Inventors:
• **GARDING, Jonas**
**114 25 Stockholm (SE)**
• **KJÄLL, Per**
**153 37 Järna (SE)**
• **LIDBERG, Pär**
**169 72 Solna (SE)**

(74) Representative: **Downing, Michael Philip et al**
**Downing IP**
**Oak House**
**Oak End Way**
**Gerrards Cross**
**Buckinghamshire SL9 8BR (GB)**

(56) References cited:
**WO-A-2005/057463      US-A- 5 373 844**
**US-A- 5 782 739**

• **LEFKOPOULOS D ET AL: "A GENERAL OPTIMIZATION PROCEDURE FOR STEREOTACTIC SMALL-BEAM MULTI-ISOCENTRIC RADIOTHERAPY" INTERNATIONAL JOURNAL OF IMAGING SYSTEMS AND TECHNOLOGY, WILEY AND SONS, NEW YORK, US, vol. 6, no. 1, 1 March 1995 (1995-03-01), pages 114-128, XP000620339 ISSN: 0899-9457**
• **WU Q JACKIE ET AL: "Morphology-guided radiosurgery treatment planning and optimization for multiple isocenters" MEDICAL PHYSICS, AIP, MELVILLE, NY, US, vol. 26, no. 10, October 1999 (1999-10), pages 2151-2160, XP012010609 ISSN: 0094-2405**
• **LI KAILE ET AL: "A constrained tracking algorithm to optimize plug patterns in multiple isocenter Gamma Knife radiosurgery planning" MEDICAL PHYSICS, AIP, MELVILLE, NY, US, vol. 32, no. 10, 23 September 2005 (2005-09-23), pages 3132-3135, XP012075170 ISSN: 0094-2405**
• **FLICKINGER ET AL: "Treatment planning for gamma knife radiosurgery with multiple isocenters" INTERNATIONAL JOURNAL OF RADIATION: ONCOLOGY BIOLOGY PHYSICS, PERGAMON PRESS, US, vol. 18, June 1990 (1990-06), pages 1495-1501, XP002115837 ISSN: 0360-3016**
• **SHEPARD D M ET AL: "Inverse treatment planning for Gamma Knife radiosurgery" MEDICAL PHYSICS, AIP, MELVILLE, NY, US, vol. 27, no. 12, December 2000 (2000-12), pages 2748-2756, XP012011033 ISSN: 0094-2405**

EP 2 069 016 B1

• Bruce E. Shapiro: "Introduction to Mathematical Modeling in Mathematica", , 3 June 1998 (1998-06-03), Retrieved from the Internet: URL: http://www.bruce-shapiro.com/pair/mbln otes.pdf [retrieved on 2010-02-23]

**Description**

FIELD OF THE INVENTION

The present invention relates to treatment planning systems, particularly for use in radiotherapy and in radiosurgery.

BACKGROUND ART

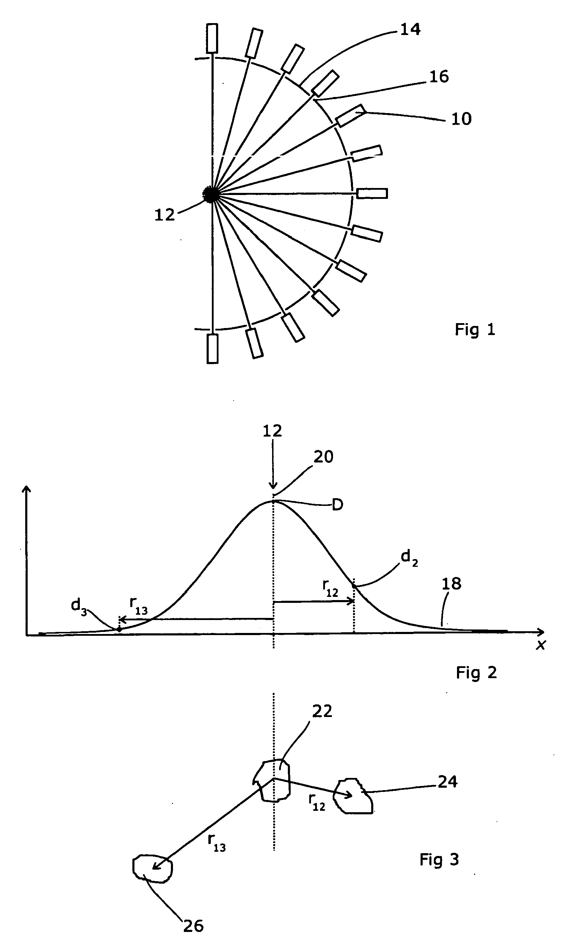

The Leksell Gamma Knife® is a well-known radiosurgical treatment device, shown illustratively in figure 1. It comprises a large number of radioactive Cobalt sources 10 set in a hemispherical array, each arranged to emit a beam of radiation toward the centre 12 of the hemisphere. A generally hemispherical collimator 14 is also provided, provided with an aperture for each source 10, each of which therefore emit a substantially pencil-shaped beam.

At the centre 12, the beams meet and the dose rate is very high. Most other locations within the hemisphere are irradiated by only one source, or possibly no sources, and thus the dose rate is very low. Locations close to the centre 12 will be irradiated by several (although not all of the) sources, as a result of the non-zero thickness of the pencil beams. Thus, the dose distribution consists of a peak at the centre 12, which falls away with increasing distance from the centre. A dose distribution generated by one isocenter is, in the field of radiosurgery, often called a "shot".

It is becoming increasingly important to provide an effective treatment solution for multiple targets (treatment locations). The basic problem in creating a treatment plan for multiple targets is that the dose contributions of the LGK interact. That is, the dose distribution given to one target will typically contribute some dose to other targets as well.

Ccmplexities can arise when there are multiple targets that require treatment. This has to date been achieved by multiple dose applications so that successive sites are placed at the radiation focus 12 (Fig 1). As a result, each target site will receive a primary dose, i.e. that delivered when at the device centre 12, together with a background dose, i.e. that delivered incidentally while a different target site is receiving a primary dose. Care must be taken to ensure that the total dose, primary and background, does not exceed the maximum intended dose,particularly outside the target.

The current treatment planning system, Leksell GammaPlan 5.34 (LGP), does allow simultaneous planning of up to 10 different targets. LGP takes the background dose contributions into account and correctly displays the total dose to each target.

SUMMARY OF THE INVENTION

LGP does not allow a dose to be explicitly prescribed to each individual target (treatment location). Instead, a global maximum dose is prescribed, which (in practice) means the local prescription to the target in which the global dose maximum occurs. To achieve the intended dose to the other targets, LGP displays (numerically) the maximum dose occurring in each target, and allows the user to change the relative weight (proportional to irradiation time) for the radiation isocenters in each target in 1% or 10% increments. By using this method iteratively, the user may eventually approach the prescribed doses for each target.

This method of balancing dose to multiple targets has several disadvantages; it is manual and time-consuming, and forces the user to keep track of the prescription isodoses outside the treatment planning system - typically on a piece of paper. The final treatment plan does not include details of these prescribed doses, making later audit or verification difficult. The user can only find out by trial and error whether the prescription doses are in fact physically achievable, meaning that if a non-achievable prescription has been requested, the user can waste much time discovering this. The process is also unintuitive and difficult to teach to new users.

Further, the user has no direct and simple way of knowing the relative dose contributions of the local and remote isocenters (treatment locations). This information is important since a strong interaction may mean that the relation between percentage isodoses and dose gradients can differ significantly from the single target case.

The present invention seeks to address these difficulties and move towards a system that overcomes them.

The present invention therefore provides a treatment planning method as set out in claim 1.

This provides a mathematically straightforward method of deriving the necessary doses to be applied to each target (treatment location) so that, including the background doses delivered while irradiating a different target, each target receives the correct total dose.

The dose factor matrix (i.e. the interaction matrix) is preferably two-dimensional, since there will be a range of target sites, each of which needs to be considered, and each target site will have a contribution offered by its own shots and each other target site. Preferably, the matrix is square, in which case each set of dose factors for each target will include the dose factor applicable to irradiation of the same target; this number will usually be 1, for obvious reasons.

The prescription matrix is preferably one-dimensional, since it needs only contain a single numerical dose for each target.

Some locations can be omitted from the treatment planning method, for example if they are located so far from the other locations that they will not have a significant interaction with them. As the size of the dose factor matrix corresponds to the number of locations in the plurality of treatment locations, a reduction in this number will simplify at least the step of inverting the matrix. This may or may not be useful, depending on the overall size of the matrix and the proceesing budget available. Thus, we prefer that the plurality of treatment locations is a subset of a larger location set consisting of (i) the plurality of treatment locations and (ii) further locations characterized by weaker interaction, for example because they are spaced from those of the plurality of treatment locations by a distance greater than the spacing of locations within the plurality of treatment locations. Group (ii) therefore consists of locations that are spatially separated from the locations in group (i), and will usually correspond to those locations where there is no significant interaction.

The present invention also relates to a treatment planning apparatus as set out in claim 6.

The treatment planning apparatus can further comprise a display means. This can be used to display the isodose lines for a planned treatment.

Other preferred aspects of the treatment planning apparatus are as set out above.

BRIEF DESCRIPTION OF THE DRAWINGS

An embodiment of the present invention will now be described by way of example, with reference to the accompanying figures in which;

Figure 1 shows in schematic form a two-dimensional section through a Leksell Gamma Knife ("LGK");

Figure 2 shows the dose distribution obtained by an LGK; and

Figure 3 illustrates multiple targets (treament locations).

DETAILED DESCRIPTION OF THE EMBODIMENTS

Figure 1 has been described above and will not be described further.

Figure 2 shows the dose distribution 18 and how this varies with position (x) in the patient along a line passing through the geometric centre 12 of the LGK. At the centre 12, the dose reaches a peak dose D at 20. This falls off either side of the peak 20, approaching zero at large distances from the centre. Figure 2 shows a relatively low rate of decline with distance, to illustrate the point.

Figure 3 shows a case with multiple targetS (treament locations) Thus, it comprises a first target site 22, together with second and third further target sites 24, 26 spaced from the first target site by distances $r_{12}$ and $r_{13}$ respectively. Thus, when a primary dose $D_1$ is being delivered to the first target site 22, the second target site receives a dose $d_{12}$ and the third target site receives a dose $d_{13}$.

The distances $r_{12}$ and $r_{13}$ and the doses $d_{12}$ and $d_{13}$ are shown in figure 2. If the distances are not large, which will often be the case with (for example) a metastasised tumour, then $d_{12}$ and $d_{13}$ may be non-negligible and will therefore need to be taken into account when planning the primary doses $D_2$ and $D_3$ that are to be delivered to the second and third target sites 24, 26. It should also be borne in mind that the delivery of primary dose $D_2$ (for example) will also involve the delivery of background doses $d_{21}$ and $d_{23}$ to the first and third target sites 22, 26 respectively, at distances $r_{12}$ and $r_{23}$ respectively. Generally therefore, for a patient with 'n' target sites the dose delivered to each site will be made up of one primary dose and (n-1) background doses, i.e. n doses in total, all of which are different and dependent on the relative locations of the sites.

For each site, any adjustment to the primary dose to compensate for the background doses will change the background doses delivered to all other sites, and hence necessitate changing their primary doses, which in turn changes the background dose for which the adjustment was being made in the first place. As n increases therefore, the plan becomes steadily more complex.

According to the invention, the first steps of the workflow for planning the treatment of multiple targets (treatment locations) are very similar (or identical) to those involved in planning a single-target treatment. Thus, each target is initially planned in isolation, i.e. as if it were the only target and without taking into account the interaction between targets. For each target, the user selects a local isodose and a prescription dose to that isodose (e.g. 20 Gy to the 50% isodose). An "isodose" is a surface (or a line when viewed in a 2D slice) joining points of equal dose. It allows the effect of the dose distribution to be visually assessed with ease despite the fact that the actual dose varies in three dimensions. Generally, the dose distribution resembies a Gaussian distribution and therefore the rates of change of dose with position is greatest around the 60% isodose line. Clinical users therefore often seek to place an isodose between 40% and 60%, at the physical boundary of the target, to maximise the dose within the target and minimise it outside the target. A dose of 40 Gy to a reference point chosen within a target is therefore often expressed as a dose of (for example) "20Gy at the 50% isodose", since the peak dose delivered by such a distribution will be 40 Gy.

The remaining variable is of course the dose amount, reflected in the time for which the radiation is applied and expressed in the treatment planning system as a "weight" applied to each irradiation. The dose is prescribed to each target separately, as in the following example.

*Table 1: Defining the prescription doses to each target*

| Target name | Selected target isodose | Prescribed dose (Gy) to isodose | Prescribed dose to ref point |
|---|---|---|---|
| A | 50% | 20 | 40 |
| B | 40% | 10 | 25 |

To illustrate that the 50% isodose is not mandatory, the isodose chosen for target B is 40% rather than 50%.

The last column is calculated automatically from the chosen isodose and the prescription dose. The reference points have been automatically set to the points of maximum dose from the shots in each target.

The dose prescriptions can be entered before or after the local planning step since (as noted above) they are independent.

The next step is to balance the interaction between the targets. If the separation of the targets is very large so that the interaction is negligible, then the balancing is normally not necessary and the planning is now finished. However, in many cases the shots in one target will contribute significantly to the total dose in other targets, so it is necessary to balance the primary and background contribution to achieve the prescribed dose for each target. According to the present invention, this is done automatically as described later. The result may look like this:

*Table 2. Result of automatic balancing of the primary and background dose contributions for each target*

| Target name | Selected target isodose | Prescribed dose (Gy) to isodose | Prescribed dose to ref point | Dose from local shots | Dose from other targets |
|---|---|---|---|---|---|
| A | 50% | 20 | 40 | 37.23 | 2.77 |
| B | 40% | 10 | 25 | 13.83 | 11.17 |

In this example, we see that the dose to target A comes mostly from the local shots, so it is likely that the local planning is acceptable without modification.

However, for target B we see that the primary and background contributions are of roughly the same magnitude, which is an indication that it will be necessary to review the dose distribution for this target carefully. If the contribution from the other targets (in this case target A) is fairly flat, then there is not much we can do since the dose gradients are then not affected by the local contribution. However, if the background contribution adds a significant gradient, then it may be necessary to compensate by adjusting the shots to target B.

We have now achieved the prescription doses for all targets (treament locations), but if we find that the isodose lines have moved more than is acceptable, we may need to adjust the plan while considering the total dose. At this point a dose volume histogram ("DVH") can be computed and evaluated for each target, taking into account the background dose contribution.

In the above discussion, we referred to an automatic process of calculating the background contribution to overall local doses and adjusting the primary doses accordingly in order to achieve the prescribed overall local doses. This will now be described. It is, of course, a non-trivial problem since the modification of the primary dose distribution at one target affects the background dose distribution which in turn affects the overall local dose distribution at every other target; a corresponding adjustment of the primary dose distributions at those other targets then affects the dose distribution at that one target, meaning that the adjusted primary dose distribution is no longer - in principle - correct.

We define $n$ as the number of targets (treament locations), and we let each target have a fixed reference point. This is typically the point of maximum dose from the local shots, but need not necessarily be. Let $m_{ij}$ be the ratio of the dose to a particular target $i$ that results from the shot(s) delivered to target $j$. For example, if the reference point for target 3 lies on the 20% isodose line for the local shots to target 5, then $m_{35} = 0.2$.

The total dose $t_i$, to the reference point, to each target can then be expressed as follows:

$$\begin{pmatrix} t_1 \\ t_2 \\ t_3 \\ \vdots \\ t_n \end{pmatrix} = \begin{pmatrix} m_{11} & m_{12} & m_{13} & \dots & m_{1n} \\ m_{21} & m_{22} & m_{23} & \dots & m_{2n} \\ m_{31} & m_{32} & m_{33} & \dots & m_{3n} \\ \vdots & \vdots & \vdots & \ddots & \vdots \\ m_{n1} & m_{n2} & m_{n3} & \dots & m_{nn} \end{pmatrix} \begin{pmatrix} d_1 \\ d_2 \\ d_3 \\ \vdots \\ d_n \end{pmatrix}$$

where $d_i$ is the maximum dose to target $i$ from the local shots in that target. In matrix format this relation can be expressed more succinctly as

$$t = Md$$

By inverting this relation, we can directly and non-iteratively determine the required dose to be given to the local reference point by the shots in each target, to achieve a given prescription dose t:

$$d = M^{-1}t$$

This allows the system to display to the user the primary contribution $d_i$ as well as the background contribution $t_i - d_i$ to each target, as shown in Table 2. The advantage of this route is that there are established mathematical algorithms for determining the inverse of a matrix; thus, by choosing to express the problem in this form and by expressing the distance between targets as isodose levels rather than actual distances, we obtain access to a straightforward route to a solution.

In a practical implementation, some variations may be advantagous. For example, each element of *d* may be scaled by some amount, and the columns of *M* can be scaled by the inverse of this amount. This variation is mathematically equivalent to the description given above.

If the interaction between targets is strong, and the prescription doses are significantly different, it may happen that the optimum solution contains negative doses d, and theretore cannot be physically realized. This can be illustrated by the following example. The reference points $r_A$ and $r_B$ in two different targets A and B both lie on the 50% local isodose for the other target. We prescribe 20 Gy to $r_A$ and 4 Gy to $r_B$. The mathematical solution is then to give 24 Gy local dose to $r_A$, and - 8 Gy local dose to $r_B$:

$$\text{Total dose to } r_A = 24 + 0.5*(-8) \text{ Gy} = 20 \text{ Gy}$$

$$\text{Total dose to } r_B = -8 + 0.5*24 \text{ Gy} = 4 \text{ Gy}$$

Unfortunately, it is not possible to produce negative dose values, so we must settle for a non-optimal solution. This can be defined in a number of ways. One way is to search for the dose that is as close as possible to the prescription dose, while satisfying the constraints that (i) no local dose values are negative, and (ii) the total dose to each target is greater than or equal to the prescription dose. This is a constrained optimization problem, which can be solved e.g. with quadratic programming techniques. For this example, the best we can do is to give 20 Gy to A and 0 Gy to B, resulting in global doses of 20 Gy and 10 Gy to A and B respectively.

Nevertheless, the above-described treatment planning method reveals immediately that there is no realisable solution, by the presence of negative primary doses. In an iterative method, it is possible for a clinician to spend a considerable time (with a more complex plan) before giving up; even then there may be a residual doubt as to whether the plan is actually non-realisable or the clinician is simply insufficiently able.

The method described above relies on choosing a reference point for each target such that it does not depend on the dose plan for the other targets. This property allows us to find a closed-form solution (if it exists) to the problem of achieving the prescribed reference dose to each target, as described above.

While planning in local mode, a system operating according to the above description may automatically adjust the 100% reference point to be the point with the maximum contribution from the shot(s) for this target. This means that there may be dose points for which the contribution from other shots means that the effective dose is shown as being above 100%. This is mathematically correct, since the reference point for each local percentage level is the locally applied dose, but it may initially be confusing to some users.

To lessen this potential for confusion, the system can be provided with functionality for finding and displaying the true maximum point within the complete treatment volume and the dose at that point. If users prefer to display isodoses relative to this true maximum, a display mode can be provided that normalizes the percentage isodoses to that global maximum. The selected isodoses should be changed in the same way, so that no isodose lines move; thus only their labels will change (e.g. from 50% to 46%). However, in this mode, the local reference point is still used as the basis for the prescription isodoses. Otherwise, the isodose levels for different targets will be interdependent and any change to one target may necessitate adjustments to other targets in an iterative way, which will be very difficult to fully understand.

To summarize, the system can provide the following three types of isodose display:

| | |
|---|---|
| 1. Local mode: | Percentage isodoses for the local shots only. Used to plan the individual doses and to plot the isodose surfaces relative to the target site surfaces. |
| 2. Fine-tuning mode: | Percentage isodoses or absolute dose values for dose to a selected target from all shots. Percentage values are based on either the maximum from the local shots or the maximum dose to the target from all shots. Dose curves are only shown within the selected dose matrix (to avoid confusion when panning to a different target without changing the selected target - one could also imagine changing the selected target automatically) |
| 3. Global mode: | Absolute dose values for all targets, shown everywhere. This is useful for checking dose to critical structures. |

It will of course be understood that many variations may be made to the above-described embodiment without departing from the scope of the present invention.

**Claims**

**1.** A treatment planning method, comprising the steps of;
defining a plurality of treatment locations and an overall dose to be applied to each;
for each location, determining the dose distribution as a result of shots aimed at said each location, in isolation from the dose as a result of shots applied to other locations;
for each specific location, defining the background dose applied at that location by shots aimed at different locations, as a proportion of the primary dose applied at the respective different location by said shots aimed at said different locations;
applying a mathematical optimization technique to automatically balance the background dose contributions and said primary doses applied at each location, in order to obtain the prescribed overall dose to each location.

**2.** A treatment planning method according to claim 1 in which the mathematical optimization technique comprises;
constructing a dose factor matrix containing for each location, the background dose contributions arising from the primary doses applied at each other location;
inverting the dose factor matrix and multiplying it with a prescription matrix containing the overall doses to be applied to each location, thereby to obtain a resultant matrix containing a primary dose for each location.

**3.** A treatment planning method according to claim 2 in which the dose factor matrix is two-dimensional.

**4.** A treatment planning method according to claim 2 or claim 3 in which the prescription matrix is one-dimensional.

**5.** A treatment planning method according to any one of the preceding claims in which the plurality of treatment locations is a subset of a location set consisting of (i) the plurality of treatment locations and (ii) further locations spaced from those of the plurality of treatment locations by a distance greater than the spacing of locations within the plurality of treatment locations.

**6.** Treatment planning Apparatus comprising;
means for accepting details of a plurality of treatment locations and an overall dose to be applied to each;
a processing means arranged to

i. for each location, determining the dose distribution resulting from shots aimed at said each location, in isolation from the dose applied to other locations;

ii. define the background dose applied at said each location by shots aimed at different locations, as a proportion of the primary dose applied at the respective different location by said shots aimed at said different location;

iii. construct a dose factor matrix containing, for each location, the background doses arising from said primary dose applied at each other location;

iv. invert the dose factor matrix and multiply it with a prescription matrix containing the overall doses to be applied to each location, thereby to obtain a resultant matrix containing a primary dose for each location; and

an output means arranged to make available the resultant matrix.

7. Treatment Planning Apparatus according to claim 6 further comprising a display means.

8. Treatment Planning Apparatus according to claim 7 arranged to display the isodose lines for a planned treatment.

9. Treatment Planning Apparatus according to any one of claims 6 to 8 in which the dose factor matrix is two-dimensional.

10. Treatment Planning Apparatus according to any one of claims 6 to 9 in which the prescription matrix is one-dimensional,

11. Treatment Planning Apparatus according to any one of claims 6 to 10 in which the plurality of treatment locations is a subset of a location set consisting of (i) the plurality of treatment locations and (ii) further locations having a lesser interaction with the locations of the plurality of treatment locations.

12. Treatment Planning Apparatus according to claim 11 in which the further locations are spaced from those of the plurality of treatment locations by a distance greater than the spacing of locations within the plurality of treatment locations.

**Patentansprüche**

1. Behandlungsplanungsverfahren, das die folgenden Schritte umfasst:

Definieren mehrerer Behandlungsstellen und einer auf jede Stelle anzuwendenden Gesamtdosis;

für jede Stelle das Bestimmen der Dosisverteilung als Ergebnis von auf jede Stelle gezielten Schüssen, in Isolation von der Dosis als Ergebnis von auf andere Stellen angewendeten Schüssen;

für jede spezifische Stelle das Definieren der von auf verschiedene Stellen gezielten Schüsse auf diese Stelle angewendeten Hintergrunddosis als Anteil der von dem auf die verschiedenen Stellen gezielten Schüsse auf die jeweilige verschiedene Stelle angewendeten Primärdosis;

Anwenden einer mathematischen Optimierungstechnik zum automatischen Ausgleichen der Hintergrunddosisbeiträge und der Primärdosen, die auf jede Stelle angewendet sind, um die vorgeschriebene Gesamtdosis zu jeder Stelle zu erhalten.

2. Behandlungsplanungsverfahren nach Anspruch 1, bei dem die mathematische Optimierungstechnik Folgendes umfasst:

Konstruieren einer Dosisfaktormatrix, die für jede Stelle die Hintergrunddosisbeiträge enthält, die sich aus den an jeder anderen Stelle angewendeten Primärdosen ergeben;

Invertieren der Dosisfaktormatrix und sie mit einer Rezeptmatrix multiplizieren, die die auf jede Stelle anzuwendenden Gesamtdosen enthält, um dadurch eine resultierende Matrix zu erhalten, die eine Primärdosis für jede Stelle enthält.

3. Behandlungsplanungsverfahren nach Anspruch 2, bei dem die Dosisfaktormatrix zweidimensional ist.

4. Behandlungsplanungsverfahren nach Anspruch 2 oder 3, bei dem die Rezeptmatrix eindimensional ist.

5. Behandlungsplanungsverfahren nach einem der vorhergehenden Ansprüche, bei dem die mehreren Behandlungsstellen eine Teilmenge einer Stellenmenge sind, die aus (i) den mehreren Behandlungsstellen und (ii) weiteren Behandlungsstellen, die von jenen der mehreren Behandlungsstellen um einen Abstand beabstandet sind, der größer ist als die Beabstandung von Stellen innerhalb der mehreren Behandlungsstellen, besteht.

6. Behandlungsplanungsvorrichtung, die Folgendes umfasst:

Mittel zum Annehmen von Details von mehreren Behandlungsstellen und einer auf jede anzuwendenden Gesamtdosis;
ein Verarbeitungsmittel, das ausgelegt ist, um

I. für jede Stelle die sich aus auf jede Stelle gezielten Schüssen ergebende Dosisverteilung zu bestimmen, in Isolation der auf andere Stellen angewendeten Dosis;
II. die auf jede Stelle durch auf verschiedene Stellen gezielte Schüsse angewendete Hintergrunddosis als ein Anteil der auf die jeweilige andere Stelle durch die auf die verschiedenen Stellen gezielten Schüsse angewendeten Primärdosis zu definieren;
III. eine Dosisfaktormatrix zu konstruieren, die für jede Stelle die Hintergrunddosen enthält, die sich aus der auf jede andere Stelle angewendeten Primärdosis ergeben;
IV. die Dosisfaktormatrix zu invertieren und sie mit einer Rezeptmatrix zu multiplizieren, die die auf jede Stelle anzuwendenden Gesamtdosen enthält, um dadurch eine resultierende Matrix zu erhalten, die eine Primärdosis für jede Stelle enthält; und

ein Ausgabemittel, das ausgelegt ist, um die resultierende Matrix verfügbar zu machen.

7. Behandlungsplanungsvorrichtung nach Anspruch 6, weiterhin umfassend ein Displaymittel.

8. Behandlungsplanungsvorrichtung nach Anspruch 7, ausgelegt zum Anzeigen der Isodosislinien für eine geplante Behandlung.

9. Behandlungsplanungsvorrichtung nach einem der Ansprüche 6 bis 8, bei der die Dosisfaktormatrix zweidimensional ist.

10. Behandlungsplanungsvorrichtung nach einem der Ansprüche 6 bis 9, bei der die Rezeptmatrix eindimensional ist.

11. Behandlungsplanungsverfahren nach einem der Ansprüche 6 bis 10, bei dem die mehreren Behandlungsstellen eine Teilmenge einer Stellenmenge sind, die aus (i) den mehreren Behandlungsstellen und (ii) weiteren Stellen mit einer geringeren Interaktion mit den Stellen der mehreren Behandlungsstellen besteht.

12. Behandlungsplanungsvorrichtung nach Anspruch 11, bei dem die weiteren Stellen von jenen der mehreren Behandlungsstellen um einen Abstand beabstandet sind, der größer ist als die Beabstandung von Stellen innerhalb der mehreren Behandlungsstellen.

**Revendications**

1. Procédé de planification de traitement comprenant les étapes consistant à :

définir une pluralité de positions de traitement et une dose globale à appliquer en chacune d'elles ;
pour chaque position, déterminer la répartition de la dose résultant d'injections visant chacune desdites positions, de manière isolée de la dose résultant d'injections appliquées à d'autres positions ;
pour chaque position particulière, définir la dose de fond appliquée à cette position par des injections visant différentes positions, sous la forme d'une proportion de la dose principale appliquée aux différentes positions par lesdites injections visant lesdites différentes positions ;
appliquer une technique d'optimisation mathématique pour équilibrer automatiquement les contributions de la dose de fond et desdites doses principales appliquées à chaque position, afin d'obtenir la dose globale prescrite à chaque position.

2. Procédé de planification de traitement selon la revendication 1, dans lequel la technique d'optimisation mathématique comprend :

la construction d'une matrice de facteurs de dose contenant, pour chaque position, les contributions à la dose de fond résultant des doses principales appliquées à chaque autre position ;

l'inversion de la matrice de facteurs de dose et sa multiplication par une matrice de prescription contenant les doses globales devant être appliquées à chaque position, pour ainsi obtenir une matrice résultante contenant une dose principale pour chaque position.

3. Procédé de planification de traitement selon la revendication 2, dans lequel la matrice de facteurs de dose est bidimensionnelle.

4. Procédé de planification de traitement selon la revendication 2 ou la revendication 3, dans lequel la matrice de prescription est monodimensionnelle.

5. Procédé de planification de traitement selon l'une quelconque des revendications précédentes, dans lequel la pluralité de positions de traitement est un sous-ensemble d'un ensemble de positions comprenant (i) la pluralité de positions de traitement, et (ii) d'autres positions espacées de celles de la pluralité de positions de traitement d'une distance supérieure à l'espacement des positions parmi la pluralité de positions de traitement.

6. Appareil de planification de traitement comprenant :

un moyen permettant d'accepter les détails d'une pluralité de positions de traitement et une dose globale devant être appliquée en chacune d'elles ;

un moyen de traitement conçu pour

i. pour chaque position, déterminer la répartition de la dose résultant d'injections visant chacune desdites positions, de manière isolée de la dose appliquée aux autres positions ;

ii. définir la dose de fond appliquée à chacune desdites positions par des injections visant différentes positions, sous la forme d'une proportion de la dose principale appliquée à la position respective différente par lesdites injections visant ladite position différente ;

iii. construire une matrice de facteurs de dose contenant, pour chaque position, les doses de fond résultant de ladite dose principale appliquée à chaque autre position ;

iv. inverser la matrice de facteurs de dose et la multiplier par une matrice de prescription contenant les doses globales devant être appliquées à chaque position, pour ainsi obtenir une matrice résultante contenant une dose principale pour chaque position, et

un moyen de sortie conçu pour rendre disponible la matrice résultante.

7. Appareil de planification de traitement selon la revendication 6, comprenant en outre un moyen d'affichage.

8. Appareil de planification de traitement selon la revendication 7, conçu pour afficher des courbes d'isodose pour un traitement planifié.

9. Appareil de planification de traitement selon l'une quelconque des revendications 6 à 8, dans lequel la matrice de facteurs de dose est bidimensionnelle.

10. Appareil de planification de traitement selon l'une quelconque des revendications 6 à 9, dans lequel la matrice de prescription est monodimensionnelle.

11. Appareil de planification de traitement selon l'une quelconque des revendications 6 à 10, dans lequel la pluralité de positions de traitement est un sous-ensemble d'un ensemble de positions comprenant (i) la pluralité de positions de traitement, et (ii) des positions supplémentaires produisant une moindre interaction avec les positions de la pluralité de positions de traitement.

12. Appareil de planification de traitement selon la revendication 11, dans lequel les autres positions sont espacées de celles de la pluralité de positions de traitement d'une distance supérieure à l'espacement entre les positions appartenant à la pluralité de positions de traitement.

Fig 1

Fig 2

Fig 3